# EUROPÄISCHE PATENTSCHRIFT

(11) **EP 1 495 015 B1**
(45) Veröffentlichungstag und Bekanntmachung des Hinweises auf die Patenterteilung: **12.04.2006**
(21) Anmeldenummer: 03701498.2
(22) Anmeldetag: 08.01.2003
(51) Int. Cl.: C07D 333/20, C07D 333/58, C07D 333/22, C07D 333/36, C07D 333/28, C07D 333/42, C07D 333/44, C07D 307/52, C07D 207/335, C07D 277/58, C07D 277/40, C07D 261/08, C07D 231/12, C07D 249/08, A61K 8/49

(54) **3-AMINOPHENOL-DERIVATE UND DIESE VERBINDUNGEN ENTHALTENDE FÄRBEMITTEL FÜR KERATINFASERN**
3-AMINOPHENOL DERIVATIVES AND DYES THAT CONTAIN THESE COMPOUNDS WHILE SERVING TO DYE KERATIN FIBERS
DERIVES DE 3-AMINOPHENOL ET COLORANTS POUR FIBRES A BASE DE KERATINE, CONTENANT LESDITS COMPOSES

(30) Priorität: 18.04.2002 DE 10217271
(43) Veröffentlichungstag der Anmeldung: 12.01.2005
(73) Patentinhaber: Wella Aktiengesellschaft, 64274 Darmstadt (DE)
(72) Erfinder: UMBRICHT, Gisela, CH-1723 Marly (CH); ROSATO, Franco, Jose, CH-3097 Liebefeld (CH); BRAUN, Hans-Jürgen, CH-3182 Überstorf (CH)
(86) Internationale Anmeldenummer: PCT/EP2003/000098
(87) Internationale Veröffentlichungsnummer: WO 2003/087084

(56) Entgegenhaltungen:
- EP-A- 1 153 917
- WO-A-01/85683
- WO-A-02/062783

## Beschreibung

Die vorliegende Erfindung betrifft neue in 2-Stellung substituierte 3-Aminophenol-Derivate sowie diese Verbindungen enthaltende Mittel zum Färben von Keratinfasern, insbesondere menschlichen Haaren.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenole, 1,4-Diaminobenzol und 4,5-Diamino-1-(2-hydroxyethyl)-pyrazol eingesetzt, während als Kupplersubstanzen beispielsweise Resorcin, 2-Methyl-resorcin, 1-Naphthol, 5-Amino-2-methylphenol, m-Phenylendiamine, 2-Amino-4-(2'-hydroxyethyl)amino-anisol, 1,3-Diamino-4-(2'-hydroxyethoxy)benzol und 2,4-Diamino-5-fluor-toluol zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, dass durch Kombination geeigneter Entwicklersubstanzen und Kupplersubstanzen eine breite Palette verschiedener Farbnuancen erzeugt werden kann.

Obwohl bereits eine Vielzahl von Kupplersubstanzen bekannt, ist es mit den derzeit bekannten Färbemitteln nicht möglich, die an ein Färbemittel gestellten Anforderungen in jeder Hinsicht zu erfüllen. Es besteht daher weiterhin ein Bedürfnis nach neuen Kupplersubstanzen, welche die vorgenannten Anforderung in besonderem Masse erfüllen.

Es wurde nunmehr gefunden, dass bestimmte 3-Aminophenol-Derivate gemäß der allgemeinen Formel (I) die an Kupplersubstanzen gestellten Anforderungen in besonders hohem Masse erfüllen und mit den meisten bekannten Entwicklersubstanzen farbstarke, außerordentlich lichtechte und waschechte Farbnuancen ergeben.

Gegenstand der vorliegenden Erfindung sind daher neue 3-Aminophenol-Derivate der Formel (I), oder deren physiologisch verträglichen wasserlösliche Salze, 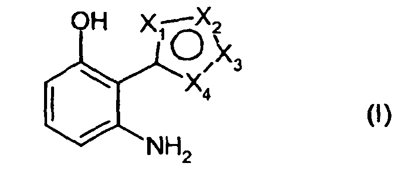 wobei **X1, X2, X3** und **X4** unabhängig voneinander gleich Schwefel, Stickstoff, N-R1, Sauerstoff, C-R2, C-R3, C-R4 oder C-R5 sind, unter der Bedingung, dass mindestens einer und höchstens drei der Reste **X1** bis **X4** gleich Stickstoff, N-R1, Schwefel oder Sauerstoff sind und wenn es mehrere Heteroatome gibt höchstens einer der Reste **X1** bis **X4** gleich Schwefel, N-R1 oder Sauerstoff ist; **R1** gleich Wasserstoff, einer C₁-C₆₋Alkylgruppe, einer Phenylgruppe, einer (C₂-C₄)-Hydroxyalkylgruppe oder einerAcetylgruppe ist; **R2, R3, R4** und **R5** unabhängig voneinander Wasserstoff, ein Halogenatom (F, Cl, Br, J), eine Cyanogruppe, eine C₁-C₆₋Alkylgruppe, eine C₁-C₄-Alkoxygruppe, eine C₁-C₄-Alkythiogruppe, eine Mercaptogruppe, eine Nitrogruppe, eine Aminogruppe, eine C₁-C₄₋Alkylamino-gruppe, eine Di(C₁-C₄)alkylaminogruppe, eine Hydroxy(C₂₋C₄)alkyl-aminogruppe, eine Di(hydroxy(C₂-C₄)alkyl)aminogruppe, eine (Dihydroxy-(C₃-C₄)alkyl)-aminogruppe, eine Trifluoromethylgruppe, eine Acetylgruppe, eine Trifluoroacetylgruppe, eine Formylgruppe, eine Trimethylsilylgruppe, eine (C₁-C₄)-Hydroxyalkylgruppe oder eine (C₂-C₄)-Dihydroxyalkylgruppe darstellen, oder es gilt (i) X1=CH, X2=CR3, X3=CR4 und X4=Schwefel, wobei die Reste R3 und R4 gemeinsam mit dem Restmolekül einen annelierten unsubstituierten Benzolring bilden, oder (ii) X4=CH, X1=CR2, X2=CR3 und X3=Schwefel, wobei die Reste R2 und R3 gemeinsam mit dem Restmolekül einen annelierten unsubstituierten Benzolring bilden.
Als Verbindungen der Formel (I) können beispielweise genannt werden: 3-Amino-2-(3-thienyl)phenol, 3-Amino-2-(3-furyl)phenol, 3-Amino-2-(pyrrol-3-yl)phenol, 3-Amino-2-(1-methyl-1 H-pyrrol-3-yl)phenol, 3-Amino-2-(1,3-thiazol-2-yl)phenol, 3-Amino-2-(1,3-thiazol-5-yl)phenol, 3-Amino-2-(2-thienyl)phenol, 3-Amino-2-(2-furyl)phenol, 3-Amino-2-(pyrrol-2-yl)-phenol, 3-Amino-2-(1-methyl-1 H-pyrrol-2-yl)phenol, 3-Amino-2-(2-chlor-3-thienyl)-phenol, 3-Amino-2-(2-methyl-3-thienyl)phenol, 3-Amino-2-(benzo[b]thiophen-2-yl)-phenol, 3-Amino-2-(5-phenyl-2-thienyl)phenol, 3-Amino-2-(2-nitro-3-thienyl)phenol, 3-Amino-2-(2-amino-3-thienyl)phenol, 3-Amino-2-(2-acetyl-3-thienyl)phenol, 3-Amino-2-(2-formyl-3-thienyl)-phenol, 3-Amino-2-(4-chlor-3-thienyl)phenol, 3-Amino-2-(4-methyl-3-thienyl)phenol, 3-Amino-2-(4-nitro-3-thienyl)phenol, 3-Amino-2-(4-amino-3-thienyl)phenol, 3-Amino-2-(4-acetyl-3-thienyl)phenol, 3-Amino-2-(4-formyl-3-thienyl)phenol, 3-Amino-2-(5-chlor-3-thienyl)phenol, 3-Amino-2-(5-methyl-3-thienyl)phenol, 3-Amino-2-(5-phenyl-3-thienyl)phenol, 3-Amino-2-(5-nitro-3-thienyl)phenol, 3-Amino-2-(5-acetyl-3-thienyl)phenol, 3-Amino-2-(5-amino-3-thienyl)phenol, 3-Amino-2-(5-formyl-3-thienyl)-phenol, 3-Amino-2-(benzo[b]thiophen-3-yl)-phenol, 3-Amino-2-(5-formyl-3-furyl)phenol, 3-Amino-2-(3-chlor-2-thienyl)phenol, 3-Amino-2-(3-methyl-2-thienyl)phenol, 3-Amino-2-(3-nitro-2-thienyl)phenol, 3-Amino-2-(3-amino-2-thienyl)phenol, 3-Amino-2-(3-acetyl-2-thienyl)-phenol, 3-Amino-2-(3-formyl-2-thienyl)phenol, 3-Amino-2-(4-chlor-2-thienyl)phenol, 3-Amino-2-(4-methyl-2-thienyl)phenol, 3-Amino-2-(4-nitro-2-thienyl)phenol, 3-Amino-2-(4-amino-2-thienyl)phenol, 3-Amino-2-(4-acetyl-2-thienyl)phenol, 3-Amino-2-(4-formyl-2-thienyl)phenol, 3-Amino-2-(5-chlor-2-thienyl)phenol, 3-Amino-2-(5-methyl-2-thienyl)phenol, 3-Amino-2-(5-nitro-2-thienyl)phenol, 3-Amino-2-(5-amino-2-thienyl)phenol, 3-Amino-2-(5-acetyl-2-thienyl)-phenol, 3-Amino-2-(5-formyl-2-thienyl)phenol, 3-Amino-2-(5-formyl-2-furyl)phenol, 3-Amino-2-(5-nitro-1,3-thiazol-2-yl)phenol, 3-Amino-2-(5-amino-1,3-thiazol-2-yl)phenol, 3-Amino-2-(2-nitro-1,3-thiazol-5-yl)phenol, 3-Amino-2-(2-amino-1,3-thiazol-5-yl)phenol, 3-Amino-2-(3,5-dimethylisoxazol-4-yl)-phenol, 3-Amino-2-(3,5-dimethyl-1 H-pyrazol-4-yl)phenol und 3-Amino-2-(5-nitro-4H-1,2,4-triazol-3-yl)phenol sowie deren physiologisch verträglichen wasserlösliche Salze.

Bevorzugt sind Verbindungen der Formel (I) in denen gilt:
(i) **X2** ist gleich Schwefel, und **X1, X3** und **X4** sind gleich C-R2, C-R4 und C-R5 oder (ii) **X1** ist gleich Schwefel und **X2, X3** und **X4** sind gleich C-R3, C-R4 und C-R5.

Besonders bevorzugt sind die folgenden Verbindungen der Formel (I): 3-Amino-2-(3-thienyl)phenol, 3-Amino-2-(2-thienyl)phenol, 3-Amino-2-(benzo[b]thiophen-3-yl)-phenol und 3-Amino-2-(5-phenyl-2-thienyl)-phenol sowie deren physiologisch verträglichen wasserlösliche Salze.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen 3-Aminophenol-Derivate der Formel (I) kann unter Verwendung von literaturbekannten Syntheseverfahren erfolgen, beispielsweise
**a**) durch eine Tetrakis(triphenylphospin)palladium(0) katalysierte Kupplung eines halogensubstituierten 3-Aminophenol der Formel (IIa) mit einem Borsäurederivat der Formel (IIIa) und anschließende Abspaltung der für die Kupplungsreaktion erforderlichen Schutzgruppen und Reduktion einer gegebenenfalls vorhandenen Nitrogruppe; 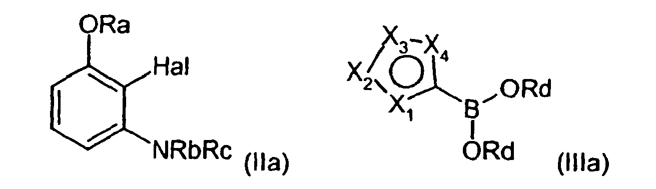 oder
**b)** durch eine Tetrakis(triphenylphospin)palladium(0) katalysierte Kupplung eines geeigneten substituierten 3-Aminophenol-borsäurederivates der Formel (IIb) mit einer halogensubstituierten aromatischen Verbindung der Formel (IIIb) und anschließende Abspaltung der für die Kupplungsreaktion erforderlichen Schutzgruppen und Reduktion einer gegebenenfalls vorhandenen Nitrogruppe; 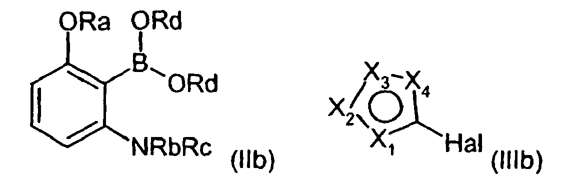
wobei die Restgruppen in den Formeln (IIa), (IIb), (IIIa) und (IIIb) die folgende Bedeutung haben:
**Ra** steht für eine Schutzgruppe, wie sie zum Beispiel in dem Kapitel "Protective Groups" in Organic Synthesis, Kapitel 3, Wiley Interscience, 1991 beschrieben wird;
**Rb** und **Rc** stehen unabhängig voneinander für Wasserstoff, eine Schutzgruppe, wie sie zum Beispiel in dem Kapitel "Protective Groups" in Organic Synthesis, Kapitel 7, Wiley Interscience, 1991 beschrieben wird, oder Rb und Rc bilden gemeinsam mit dem N-Atom eine Nitrogruppe;
**Rd** ist gleich Wasserstoff oder die beiden Rd-Reste bilden gemeinsam mit der O-B-O-Gruppe einen unsubstituierten oder substituierten fünfgliedrigen oder sechsgliedrigen cycloaliphatischen Ring;
**Hal** ist gleich F, Cl, Br oder J; und
**X1, X2, X3** und **X4** haben die in der Formel (I) angegebene Bedeutung.

Die 3-Aminophenol-Derivate der Formel (I) sind gut in Wasser löslich und ermöglichen Färbungen mit ausgezeichneter Farbintensität und Farbechtheit, insbesondere was die Lichtechtheit, Waschechtheit und Reibechtheit anbetrifft. Sie weisen weiterhin eine ausgezeichnete Lagerstabilität, insbesondere als Bestandteil der nachfolgend beschriebenen Oxidationsfärbemitteln, auf.

Ein weiterer Gegenstand der vorliegende Erfindung ist daher ein Mittel zur Färbung von Keratinfasern, wie zum Beispiel Wolle, Pelzen, Federn oder Haaren und insbesondere menschlichen Haaren, auf der Basis einer Entwicklersubstanz-Kupplersubstanz-Kombination, welches dadurch gekennzeichnet ist, dass es mindestens ein 3-Aminophenol-Derivat der Formel (I) oder dessen physiologisch verträglichen wasserlösliche Salze enthält.

Die 3-Aminophenol-Derivate der Formel (I) sind in dem erfindungsgemäßen Färbemittel in einer Gesamtmenge von etwa 0,005 bis 20 Gewichtsprozent enthalten, wobei eine Menge von etwa 0,01 bis 5 Gewichtsprozent und insbesondere 0,1 bis 2,5 Gewichtsprozent bevorzugt ist.

Als Entwicklersubstanzen kommen vorzugsweise 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(2-thienyl)benzol, 1,4-Diamino-2-(3-thienyl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)-amino]-anilin, 4-[Di(2-hydroxyethyl)amino]-anilin, 4-[Di(2-hydroxyethyl)-amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(1-hydroxyethyl)-benzol, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1 H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1 H-pyrazol, 4,5-Diamino-1-methyl-1 H-pyrazol, 2-Amino-phenol, 2-Amino-6-methyl-phenol, 2-Amino-5-methyl-phenol und 1,2,4-Trihydroxy-benzol in Betracht.

Weiterhin kann das erfindungsgemäße Färbemittel zusätzlich zu den Verbindungen der Formel (I) noch weitere bekannte Kupplersubstanzen, beispielsweise N-(3-Dimethylamino-phenyl)-harnstoff, 2,6-Diamino-pyridin, 2-Amino-4-[(2-hydroxyethyl)amino]-anisol, 2,4-Diamino-1-fluor -5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion, enthalten.

Die Kupplersubstanzen und Entwicklersubstanzen können in dem erfindungsgemäßen Färbemittel jeweils einzeln oder im Gemisch miteinander enthalten sein, wobei die Gesamtmenge an Kuppler substanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Überschuß oder Unterschuß vorhanden sind.

Weiterhin kann das erfindungsgemäße Färbemittel zusätzlich andere Farbkomponenten, wie zum Beispiel 6-Amino-2-methylphenol und 2-Amino-5-methylphenol, sowie ferner übliche synthetische oder natürliche direktziehende Farbstoffe, beispielsweise Pflanzenfarbstoffe oder synthetische direktziehende Farbstoffe aus der Gruppe der sauren oder basischen Farbstoffe (beispielsweise die in der WO 95/15144 oder EP-OS 0 850 638 beschriebenen kationischen Farbstoffe), der Triphenylmethanfarbstoffe, der aromatischen Nitrofarbstoffe, der Azofarbstoffe und der Dispersionsfarbstoffe, enthalten. Die erfindungsgemäßen Färbemittel können diese Farbkomponenten in einer Menge von etwa 0,1 bis 4 Gewichtsprozent enthalten.

Selbstverständlich können die zusätzlichen Kupplersubstanzen sowie die Entwicklersubstanzen und die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie höhere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 30 Gewichtsprozent und die Pflegestoffe in einer Konzentration von etwa 0,1 bis 5 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch Aminosäuren und/oder organische Amine, zum Beispiel Monoethanolamin und Triethanolamin, sowie anorganische Basen, wie zum Beispiel Natriumhydroxid und Kaliumhydroxid Verwendung finden. Für eine pH-Einstellung im sauren Bereich kommen anorganische oder organische Säuren, zum Beispiel Phosphorsäure, Essigsäure, Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Färbemittel mit einem Gehalt an 3-Aminophenol-Derivaten der Formel (I) als Kupplersubstanz ermöglicht Färbungen mit ausgezeichneter Farbechtheit, insbesondere was die Lichtechtheit, Waschechtheit und Reibeechtheit anbetrifft. Hinsichtlich der färberischen Eigenschaften bietet das erfindungsgemäße Färbemittel je nach Art und Zusammensetzung der Farbkomponenten eine breite Palette verschiedener Farbnuancen, welche sich von blonden über braune, purpume, violette bis hin zu blauen und schwarzen Farbtönen erstreckt. Hierbei zeichnen sich die Farbtöne durch ihre besondere Farbintensität aus. Die sehr guten färberischen Eigenschaften des Färbemittels gemäß der vorliegenden Anmeldung zeigen sich weiterhin darin, dass dieses Mittel insbesondere auch eine Anfärbung von ergrauten, chemisch nicht vorgeschädigten Haaren problemlos und mit guter Deckkraft ermöglicht.

Die nachfolgenden Beispiele sollen den Gegenstand der Erfindung näher erläutern, ohne ihn darauf zu beschränken.

### Beispiele

### Beispiel 1 bis 6: Synthese von 3-Aminophenol-Derivaten der allgemeinen Formel (I)

### A. Synthese von 2-Brom-3-nitrophenol

Zu einer Suspension von 23,1 g (150 mmol) 2-Amino-3-nitrophenol in 40 ml einer 48% Bromwasserstoffsäure und 12 ml Wasser gibt man bei 0 °C langsam eine Lösung von 10,5 g (152 mmol) Natriumnitrit in 40 ml Wasser zugetropft. Das Gemisch wird anschließend 15 Minuten lang bei 0 °C gerührt. Dann gibt man tropfenweise eine Suspension von 22,5 g Kupfer(I)-bromid (Cu₂Br₂; 78,7 mmol) in 75 ml einer 48% Bromwasserstoffsäure hinzu und rührt das Gemisch während 15 Minuten bei 0 °C und anschliessend noch 1 Stunde bei 100 °C. Anschließend wird das Reaktionsgemisch erneut auf ca. 5°C gekühlt, filtriert und der Filtrationsrückstand mit wenig Wasser gewaschen. Dieser Rückstand wird in Essigsäureethylester über Kieselgel filtriert und anschliessend das Lösungsmittel im Vakuum zur Trockenheit eingedampft.
Es werden 32,2 g (98%) 2-Brom-3-nitrophenol erhalten. Das erhaltene Rohprodukt wird ohne weitere Reinigung in der nächsten Stufe eingesetzt. ¹H-NMR (300 MHz, CDCl₃): δ= 7,47 ppm (dd, J = 1,5 Hz/7,8 Hz, 1 H, CH); 7,37 ppm (t, J = 8,1 Hz, 1H. CH); 7,26 ppm (dd, J = 1,5 Hz/8,1 Hz, 1H. CH); 6,08 ppm (s, 1 H, OH).
EI-MS: 219/217 [M⁺] (40); 161/159 [M⁺-C-NO₂] (24)

### B. Synthese von 2-Brom-1-(methoxymethoxy)-3-nitrobenzol

15 g (69 mmol) 2-Brom-3-nitrophenol aus Stufe A wird in 150 ml trockenem Acetonitril gelöst und bei 0°C portionsweise mit 3,5 g (117 mmol) einer 80%igen Natriumhydridsuspension versetzt. Anschliessend wird eine Lösung von 6,1 g (75 mmol) Chlormethoxymethan in 50 ml trockenem Acetonitril zugegeben. Nach beendeter Zugabe wird über Nacht bei Raumtemperatur gerührt. Um überschüssiges Natriumhydrid zu zersetzen werden 10 ml Ethanol zugegeben, die Reaktionsmischung filtriert und das Filtrat am Rotationsverdampfer im Vakuum zur Trockenheit eingedampft. Das erhaltene Rohprodukt kann ohne weitere Reinigung in die nächste Stufe eingesetzt werden.
Es werden 14,6 g (81 % der Theorie) an 2-Brom-1-(methoxymethoxy)-3-nitrobenzol als braunes Öl erhalten.
¹H-NMR (300 MHz, DMSO): δ = 7,60-7,48 ppm (m, 3H, arom.-CH); 5,41 ppm (s, 2H, CH₂); 3,44 ppm (s. 3H, CH₃).
MS (API-ES Neg.): 218/216 [M-H]⁻ (100)

### C. Synthese der 3-Aminophenole der Formel (I)

0,26 g (1,0 mmol) 2-Brom-1-(methoxymethoxy)-3-nitrobenzol aus Stufe B und 1,5 mmol des entsprechenden Borsäurederivates werden unter Argon in 5 ml 1,2-Dimethoxyethan gelöst. Anschließend werden 0,18 g (0,15 mmol) Tetrakis-(triphenylphosphin)-palladium(0)komplex und 0,8 ml einer wässrigen 2N Kaliumcarbonat-Lösung zugegeben und die Reaktionsmischung auf 100 °C erwärmt. Nach Beendigung der Reaktion wird die Reaktionsmischung in 10 ml Essigsäureethylester gegossen, die organische Phase mit 5 ml Wasser extrahiert, die wässrige Phase noch zweimal mit Essigsäureethylester rückextrahiert, die vereinigten organischen Phasen sodann mit Magnesiumsulfat getrocknet und das Lösungsmittel am Rotationsverdampfer abdestilliert. Der Rückstand wird an Kieselgel mit Heptan/Essigsäureethylester gereinigt.

Das so erhaltene Produkt wird in 5 ml Ethanol gelöst und in Gegenwart von etwa 50 mg Palladium (10% auf Aktivkohle) bei Raumtempereratur und unter Normaldruck mit Wasserstoffgas hydriert. Nach Beendigung der Reaktion wird das Reaktionsprodukt durch Cellite® filtriert und aufkonzentriert. Der so erhaltene Rückstand wird mit 1 ml einer 2,9molaren ethanolischen Salzsäurelösung oder mit einer 4molaren Salzsäure in Dioxan versetzt. Die Reaktionsmischung wird etwa eine Stunde bei Raumtemperatur gerührt. Nach Beendigung der Reaktion wird der Niederschlag abfiltriert, mit Ethanol (oder Dioxan) gewaschen und sodann getrocknet. Falls keine Ausfällung stattfindet, kann das Lösungsmittel am Rotationsverdampfer abgedampft werden.

### 1. 3-Amino-2-(3-thienyl)phenol-hydrochlorid

Verwendetes Borsäurederivat: 3-Thiophenborsäure
Ausbeute: 0,165 g (74% der Theorie)
¹H-NMR (300 MHz, DMSO): δ = 9,90 ppm (s, 1H, OH); 7,68-7,63 ppm (m, 2H, arom. H); 7,23-7,17 ppm (m, 2H, arom. H); 6,88 ppm (t, J= 6,9 Hz, 2H, arom. H); 3,8 - 3,3 ppm (br, NH₃⁺).
MS (API-ES Pos.): 192 [M+H]⁺ (20).

### 2. 3-Amino-2-(benzo[b]thiophen-3-yl)-phenol-hydrochlorid

Verwendetes Borsäurederivat: 1-Benzthiophen-3-yl-borsäure
Ausbeute: 0,050 g (18% der Theorie)
MS (API-ES Pos.): 242 [M+H]⁺ (50).

### 3. 3-Amino-2-(2-thienyl)phenol-hydrochlorid

Verwendetes Borsäurederivat: 2-Thiophenborsäure
Ausbeute: 0,150 g (26% der Theorie)
¹H-NMR (300 MHz, DMSO): δ = 10,39 (s, br, 1H, OH); 6,43 ppm (d, J=4,41 Hz, 1 H); 6,13 ppm (t, J=1,7, J=8,17 Hz, 1 H); 6,00 ppm (dd, J=3,6 und 5,07 Hz, 1 H); 5,89 ppm (d, J=2,6 Hz, 1 H); 5,74 ppm (dd, J=8,4 und 10,7 Hz, 2H); 3,7 - 3,3 ppm (s, br, NH₃⁺); 2,54 (s, 3H, CH3).
ESI-MS: 192 [M+1]⁺ (85)
CHN-Analyse:

| (C₁₀H₉NOS*HCl) | % C | % H | % N | %Cl | %S |
|---|---|---|---|---|---|
| berechnet: | 52,75 | 4,43 | 6,15 | 15,57 | 14,08 |
| gefunden: | 52,50 | 4,40 | 6,10 | 15,50 | 13,60 |

### 4. 3-Amino-2-(benzo[b]thiophen-2-yl)-phenol-hydrochlorid

Verwendetes Borsäurederivat: 1-Benzthiophen-2-ylborsäure
Ausbeute: 0,008 g (14% der Theorie)
MS (API-ES Pos.): 242 [M+H]⁺(50).

### 5. 3-Amino-2-(5-phenyl-2-thienyl)-phenol-hydrochlorid

Verwendetes Borsäurederivat: 5-Phenyl-2-thienylborsäure
Ausbeute: 0,048 g (16% der Theorie)
MS (API-ES Neg.): 266 [M-H]⁻ (100).

### 6. 3-Amino-2-(3,5-dimethyl-isoxazol-4-yl)-phenol-hydrochlorid

Verwendetes Borsäurederivat: 3,5-Dimethyl-4-isoxazolylborsäure
Ausbeute: 0,070 g (30% der Theorie)
MS (API-ES Pos.): 227 [M+Na]⁺ (100).

### Beispiele 7 bis 12: Haarfärbemittel

Es werden Haarfärbelösungen der folgenden Zusammensetzung hergestellt:

| | |
|---|---|
| 1,25 mmol | Substanz der Formel (I) gemäß Tabelle 1 |
| 1,25 mmol | Entwicklersubstanz gemäß Tabelle 1 |
| 10,0 g | Laurylethersulfat (28prozentige wässrige Lösung) |
| 9,0 g | Ammoniak (22prozentige wässrige Lösung) |
| 7,8 g | Ethanol |
| 0,3 g | Ascorbinsäure |
| 0,3 g | Ethylendiaminotetraessigsäure-Dinatriumsalz-Hydrat |
| ad 100,0 g | Wasser, vollentsatzt |

10 g der vorstehenden Färbelösung werden unmittelbar vor der Anwendung mit 10 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefasst.

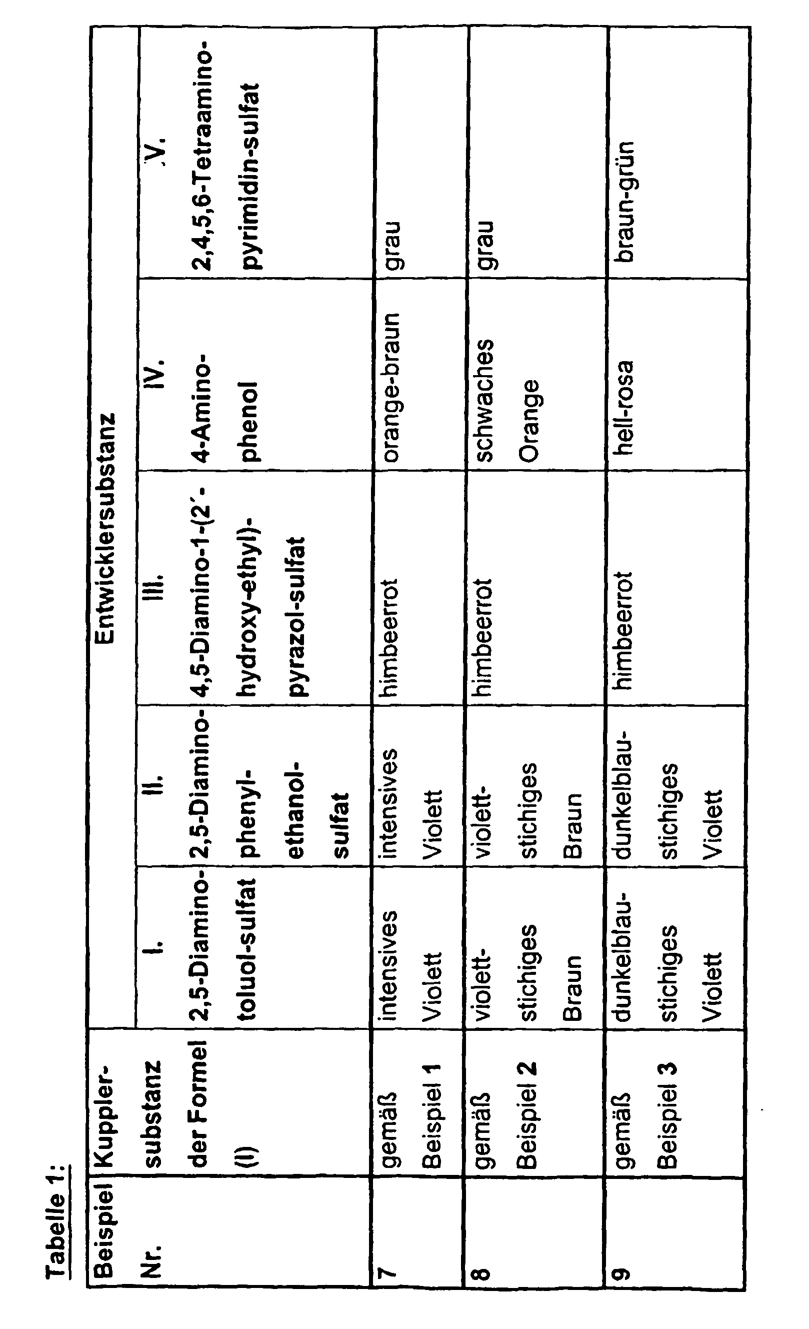

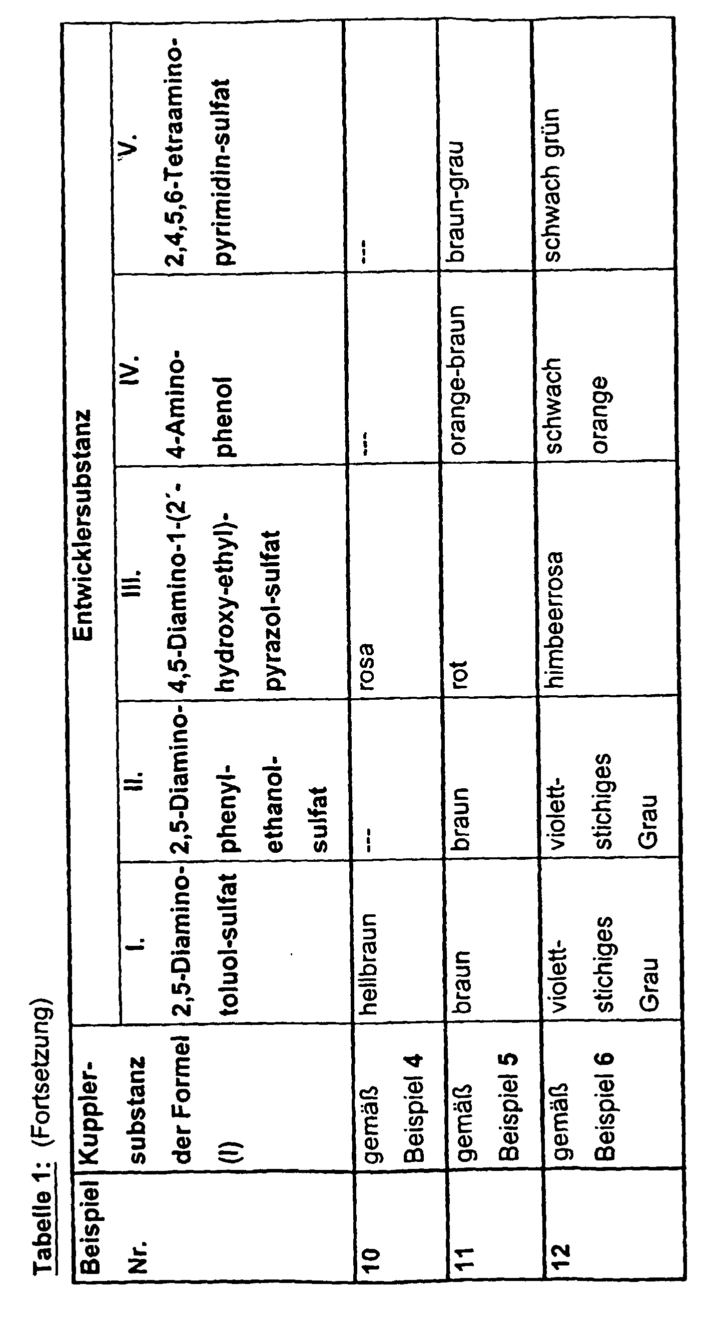

### Beispiel 13 bis 24: Haarfärbemittel

Es werden Haarfärbelösungen der folgenden Zusammensetzung hergestellt:

| | |
|---|---|
| X g | 3-Aminophenol-Derivat der Formel (I) |
| | (Kupplersubstanz **K1** bis **K2** gemäss Tabelle 4) |
| U g | Entwicklersubstanz **E8** bis **E15** gemäß Tabelle 2 |
| Y g | Kupplersubstanz **K11** bis **K36** gemäß Tabelle 4 |
| 10,0 g | Laurylethersulfat (28prozentige wässrige Lösung) |
| 9,0 g | Ammoniak (22prozentige wässrige Lösung) |
| 7,8 g | Ethanol |
| 0,3 g | Ascorbinsäure |
| 0,3 g | Ethylendiaminotetraessigsäure-Dinatriumsalz-Hydrat |
| ad 100,0 g | Wasser, vollentsalzt |

30 g der vorstehenden Färbelösung werden unmittelbar vor der Anwendung mit 30 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabellen 5 und 6 zusammengefasst.

**Tabelle 2:**

| **Entwicklersubstanzen** | |
|---|---|
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol-dihydrochlorid |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3**

| **Direktziehende Farbstoffe** | |
|---|---|
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K1** | 3-Amino-2-(3-thienyl)phenol |
| **K2** | 3-Amino-2-(2-thienyl)phenol |
| | |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K18** | N-(3-Dimethylamino)phenylharnstoff |
| **K19** | 1,3-Bis(2,4-diaminophenoxy)propan-tetrahydrochlorid |
| **K21** | 3-Amino-phenol |
| **K22** | 5-Arnino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzol*HCl |
| **K36** | 2-Amino-5-methyl-phenol |

**Tabelle 5: Haarfärbemittel**

| **Beispiel Nr.** | **13** | **14** | **15** | **16** | **17** | **18** |
|---|---|---|---|---|---|---|
| **Farbstoffe** | (Farbstoffmenge in Gramm) | | | | | |
| **K1** | 0,10 | 0,12 | 0,05 | 0,07 | 0,10 | 0,12 |
| **E8** | 0,30 | | | | | |
| **E9** | | | | | 0,25 | 0,20 |
| **E10** | | | | | | 0,10 |
| **E15** | | 0,25 | 0,30 | 0,25 | | |
| **K12** | | | 0,05 | | | |
| **K13** | | | | 0,05 | | |
| **K21** | 0,05 | | | | | |
| **K22** | | 0,05 | | | | |
| **K23** | | | 0,05 | 0,10 | 0,10 | 0,10 |
| **K25** | | | | | 0,10 | |
| **K31** | 0,20 | | | 0,15 | 0,10 | 0,10 |
| **K32** | | 0,20 | | 0,10 | | |
| **K33** | | | 0,20 | | | |
| **K36** | | | | | | 0,10 |
| **Färbeergebnis** | blond | blond | blond | blond | blond | blond |

**Tabelle 6: Haarfärbemittel**

| **Beispiel Nr.** | **19** | **20** | **21** | **22** | **23** | **24** |
|---|---|---|---|---|---|---|
| **Farbstoffe** | (Farbstoffmenge in Gramm) | | | | | |
| **K2** | 0,10 | 0,12 | 0,05 | 0,07 | 0,10 | 0,12 |
| **E8** | 0,30 | | | | | |
| **E9** | | | | | 0,25 | 0,20 |
| **E10** | | | | | | 0,10 |
| **E15** | | 0,25 | 0,30 | 0,25 | | |
| **K12** | | | 0,05 | | | |
| **K13** | | | | 0,05 | | |
| **K21** | 0,05 | | | | | |
| **K22** | | 0,05 | | | | |
| **K23** | | | 0,05 | 0,10 | 0,10 | 0,10 |
| **K25** | | | | | 0,10 | |
| **K31** | 0,20 | | | 0,15 | 0,10 | 0,10 |
| **K32** | | 0,20 | | 0,10 | | |
| **K33** | | | 0,20 | | | |
| **K36** | | | | | | 0,10 |
| **Färbeergebnis** | blond | blond | blond | blond | blond | blond |

### Beispiele 25 bis 30: Haarfärbemittel

Es werden Haarfärbelösungen der folgenden Zusammensetzung hergestellt:

| | |
|---|---|
| X g | 3-Aminophenol-Derivat der Formel (I) |
| | (Kupplersubstanz **K1** gemäss Tabelle 4) |
| U g | Entwicklersubstanz **E8** bis **E15** gemäß Tabelle 2 |
| Y g | Kupplersubstanz **K11** bis **K36** gemäß Tabelle 4 |
| Z g | direktziehende Farbstoffe **D2** und **D3** gemäß Tabelle3 |
| 10,0 g | Laurylethersulfat (28prozentige wässrige Lösung) |
| 9,0 g | Ammoniak (22prozentige wässrige Lösung) |
| 7,8 g | Ethanol |
| 0,3 g | Ascorbinsäure |
| 0,3 g | Ethylendiaminotetraessigsäure-Dinatriumsalz-Hydrat |
| ad 100,0 g | Wasser, vollentsalzt |

30 g der vorstehenden Färbecreme werden unmittelbar vor der Anwendung mit 30 g einer 6prozentigen Wasserstoffperoxidlösung vermischt. Anschließend wird das Gemisch auf das Haar aufgetragen. Nach einer Einwirkzeit von 30 Minuten bei 40°C wird das Haar mit Wasser gespült, mit einem handelsüblichen Shampoo gewaschen und getrocknet. Die Färbeergebnisse sind in Tabelle 7 zusammengefasst.

**Tabelle 7: Haarfärbemittel**

| **Beispiel Nr.** | **25** | **26** | **27** | **28** | **29** | **30** |
|---|---|---|---|---|---|---|
| **Farbstoffe** | (Farbstoffmenge in Gramm) | | | | | |
| **K1** | 0,60 | 1,30 | 1,15 | 0,15 | 0,15 | 0,15 |
| **E8** | 1,50 | | | | | |
| **E11** | 0,10 | | | | | |
| **E13** | | 1,60 | | | | 0,70 |
| **E14** | | | | 0,10 | 0,10 | |
| **E15** | | | 1,80 | 0,70 | 0,70 | |
| **K12** | 0,50 | | | | | |
| **K14** | 0,10 | | | | | |
| **K18** | 0,05 | | | | | |
| **K19** | 0,10 | | | | | |
| **K23** | | | 0,05 | 0,10 | 0,10 | 0,10 |
| **K24** | 0,15 | | | | | |
| **K31** | 0,90 | 1,10 | 1,10 | 0,40 | 0,40 | 0,40 |
| **K34** | 0,10 | | | | | |
| **D2** | | | | 0,10 | 0,10 | 0,10 |
| **D3** | | | | 0,05 | 0,05 | 0,05 |
| **Färbeergebnis** | schwarz | schwarz | schwarz | braun | braun | braun |

Alle in der vorliegenden Anmeldung enthaltenen Prozentangaben stellen, soweit nicht anders angegeben, Gewichtsprozente dar.

## Patentansprüche

1. 3-Aminophenol-Derivate der Formel (I), oder deren physiologisch verträglichen wasserlösliche Salze, 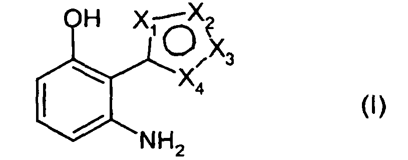 wobei **X1, X2, X3** und **X4** unabhängig voneinander gleich Schwefel, Stickstoff, N-R1, Sauerstoff, C-R2, C-R3, C-R4 oder C-R5 sind, unter der Bedingung, dass mindestens einer und höchstens drei der Reste **X1** bis **X4** gleich Stickstoff, N-R1, Schwefel oder Sauerstoff sind und wenn es mehrere Heteroatome gibt höchstens einer der Reste **X1** bis **X4** gleich Schwefel, N-R1 oder Sauerstoff ist;
**R1** gleich Wasserstoff, einer C₁-C₆-Alkylgruppe, einer Phenylgruppe, einer (C₂-C₄)-Hydroxyalkylgruppe oder einerAcetylgruppe ist;
**R2, R3, R4** und **R5** unabhängig voneinander Wasserstoff, ein Halogenatom, eine Cyanogruppe, eine C₁-C₆-Alkylgruppe, eine C₁-C₄-Alkoxygruppe, eine C₁-C₄-Alkylthiogruppe, eine Mercaptogruppe, eine Nitrogruppe, eine Aminogruppe, eine C₁-C₄-Alkylaminogruppe, eine Di(C₁-C₄)alkylaminogruppe, eine Hydroxy(C₂-C₄)alkylaminogruppe, eine Di(hydroxy(C₂-C₄)alkyl)aminogruppe, eine (Dihydroxy(C₃-C₄)alkyl)-aminogruppe, eine Trifluoromethylgruppe, eine Acetylgruppe, eine Trifluoroacetylgruppe, eine Formylgruppe, eine Trimethylsilylgruppe, eine (C₁-C₄)-Hydroxyalkylgruppe oder eine (C₂-C₄)-Dihydroxyalkylgruppe darstellen, oder es gilt (i) X1=CH, X2=CR3, X3=CR4 und X4=Schwefel, wobei die Reste R3 und R4 gemeinsam mit dem Restmolekül einen annelierten unsubstituierten Benzolring bilden, oder (ii) X4=CH, X1=CR2, X2=CR3 und X3=Schwefel, wobei die Reste R2 und R3 gemeinsam mit dem Restmolekül einen annelierten unsubstituierten Benzolring bilden.

2. 3-Aminophenol-Derivat nach Anspruch 1, **dadurch gekennzeichnet, dass** es ausgewählt ist aus 3-Amino-2-(3-thienyl)phenol, 3-Amino-2-(3-furyl)phenol, 3-Amino-2-(pyrrol-3-yl)phenol, 3-Amino-2-(1-methyl-1 H-pyrrol-3-yl)phenol, 3-Amino-2-(1,3-thiazol-2-yl)phenol, 3-Amino-2-(1,3-thiazol-5-yl)phenol, 3-Amino-2-(2-thienyl)phenol, 3-Amino-2-(2-furyl)-phenol, 3-Amino-2-(pyrrol-2-yl)phenol, 3-Amino-2-(1-methyl-1 H-pyrrol-2-yl)phenol, 3-Amino-2-(2-chlor 3-thienyl)-phenol, 3-Amino-2-(2-methyl-3-thienyl)phenol, 3-Amino-2-(benzo[b]thiophen-2-yl)-phenol, 3-Amino-2-(5-phenyl-2-thienyl)phenol, 3-Amino-2-(2-nitro-3-thienyl)phenol, 3-Amino-2-(2-amino-3-thienyl)phenol, 3-Amino-2-(2-acetyl-3-thienyl)phenol, 3-Amino-2-(2-formyl-3-thienyl)phenol, 3-Amino-2-(4-chlor-3-thienyl)phenol, 3-Amino-2-(4-methyl-3-thienyl)phenol, 3-Amino-2-(4-nitro-3-thienyl)phenol, 3-Amino-2-(4-amino-3-thienyl)phenol, 3-Amino-2-(4-acetyl-3-thienyl)-phenol, 3-Amino-2-(4-formyl-3-thienyl)phenol, 3-Amino-2-(5-chlor-3-thienyl)phenol, 3-Amino-2-(5-methyl-3-thienyl)phenol, 3-Amino-2-(5-phenyl-3-thienyl)phenol, 3-Amino-2-(5-nitro-3-thienyl)phenol, 3-Amino-2-(5-acetyl-3-thienyl)phenol, 3-Amino-2-(5-amino-3-thienyl)-phenol, 3-Amino-2-(5-formyl-3-thienyl)phenol, 3-Amino-2-(benzo[b]-thiophen-3-yl)-phenol, 3-Amino-2-(5-formyl-3-furyl)phenol, 3-Amino-2-(3-chlor-2-thienyl)phenol, 3-Amino-2-(3-methyl-2-thienyl)phenol, 3-Amino-2-(3-nitro-2-thienyl)phenol, 3-Amino-2-(3-amino-2-thienyl)phenol, 3-Amino-2-(3-acetyl-2-thienyl)-phenol, 3-Amino-2-(3-formyl-2-thienyl)phenol, 3-Amino-2-(4-chlor-2-thienyl)phenol, 3-Amino-2-(4-methyl-2-thienyl)-phenol, 3-Amino-2-(4-nitro-2-thienyl)phenol, 3-Amino-2-(4-amino-2-thienyl)phenol, 3-Amino-2-(4-acetyl-2-thienyl)phenol, 3-Amino-2-(4-formyl-2-thienyl)phenol, 3-Amino-2-(5-chlor-2-thienyl)phenol, 3-Amino-2-(5-methyl-2-thienyl)phenol, 3-Amino-2-(5-nitro-2-thienyl)phenol, 3-Amino-2-(5-amino-2-thienyl)phenol, 3-Amino-2-(5-acetyl-2-thienyl)phenol, 3-Amino-2-(5-formyl-2-thienyl)phenol, 3-Amino-2-(5-formyl-2-furyl)phenol, 3-Amino-2-(5-nitro-1,3-thiazol-2-yl)phenol, 3-Amino-2-(5-amino-1,3-thiazol-2-yl)-phenol, 3-Amino-2-(2-nitro-1,3-thiazol-5-yl)phenol, 3-Amino-2-(2-amino-1,3-thiazol-5-yl)phenol, 3-Amino-2-(3,5-dimethyl-isoxazol-4-yl)-phenol, 3-Amino-2-(3,5-dimethyl-1 H-pyrazol-4-yl)phenol und 3-Amino-2-(5-nitro-4H-1,2,4-triazol-3-yl)phenol sowie deren physiologisch verträglichen wasserlöslichen Salze.

3. 3-Aminophenol-Derivat nach Anspruch 1, **dadurch gekennzeichnet, dass** in der Formel (I) gilt:
(i) **X2** ist gleich Schwefel, und **X1, X3** und **X4** sind gleich C-R2, C-R4 und C-R5 oder (ii) **X1** ist gleich Schwefel und **X2, X3** und **X4** sind gleich C-R3, C-R4 und C-R5.

4. 3-Aminophenol-Derivat nach einem der Ansprüche 1 bis 3, **dadurch gekennzeichnet, dass** es ausgewählt ist aus 3-Amino-2-(3-thienyl)phenol, 3-Amino-2-(2-thienyl)phenol, 3-Amino-2-(benzo[b]-thiophen-3-yl)-phenol und 3-Amino-2-(5-phenyl-2-thienyl)-phenol sowie deren physiologisch verträglichen wasserlöslichen Salze.

5. Mittel zur Färbung von Keratinfasem auf der Basis einer Entwicklersubstanz-Kupplersubstanz-Kombination, **dadurch gekennzeichnet, dass** es als Kupplersubstanz mindestens ein 3-Aminophenol-Derivat der Formel (I) nach einem der Ansprüche 1 bis 4 enthält.

6. Mittel nach Anspruch 5, **dadurch gekennzeichnet, dass** das 3-Aminophenol-Derivat der Formel (I) in einer Menge von 0,005 bis 20 Gewichtsprozent enthalten ist.

7. Mittel nach Anspruch 5 oder 6, **dadurch gekennzeichnet, dass** die Entwicklersubstanz ausgewählt ist aus der Gruppe bestehend aus 1,4-Diamino-benzol, 1,4-Diamino-2-methyl-benzol, 1,4-Diamino-2,6-dimethyl-benzol, 1,4-Diamino-3,5-diethyl-benzol, 1,4-Diamino-2,5-dimethyl-benzof, 1,4-Diamino-2,3-dimethyl-benzoi, 2-Chlor-1,4-diaminobenzol, 1,4-Diamino-2-(2-thienyl)benzol, 1,4-Diamino-2-(3-thienyl)benzol, 1,4-Diamino-2-(pyridin-3-yl)benzol, 2,5-Diamino-biphenyl, 1,4-Diamino-2-methoxymethyl-benzol, 1,4-Diamino-2-amino-methylbenzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methylanilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(1-hydroxyethyl)-benzol, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methylphenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1 H-pyrazol, 4,5-Diamino-1-(1-methylethyl)-1 H-pyrazol, 4,5-Diamino-1-[(4-methylphenyl)methyl]-1 H-pyrazol, 1-[(4-Chlorphenyl)methyl]-4,5-diamino-1H-pyrazol, 4,5-Diamino-1-methyl-1 H-pyrazol, 2-Amino-phenol, 2-Amino-6-methyl-phenol, 2-Amino-5-methyl-phenol und 1,2,4-Trihydroxy-benzol.

8. Mittel nach einem der Ansprüche 5 bis 7, **dadurch gekennzeichnet, dass** es zusätzlich zu den Verbindungen der Formel (I) mindestens eine weitere bekannte Kupplersubstanzen enthält, welche ausgewählt ist aus der Gruppe bestehend aus N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2 ,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

9. Mittel nach einem der Ansprüche 5 bis 8, **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

10. Mittel nach einem der Ansprüche 5 bis 9 , **dadurch gekennzeichnet, dass** es zusätzlich mindestens einen direktziehenden Farbstoff enthält.

11. Mittel nach einem der Ansprüche 5 bis 10, **dadurch gekennzeichnet, dass** es einen pH-Wert von 6,5 bis 11,5 aufweist.

12. Gebrauchsfertiges Mittel zur oxidativen Färbung von Keratinfasern, welches in einem zum Färben geeigneten Medium mindestens eine Entwicklersubstanz und mindestens eine Kupplersubstanz sowie mindestens ein Oxidationsmittel enthält, **dadurch gekennzeichnet, dass** es als Kupplersubstanz mindestens ein 3-Aminophenol-Derivat der Formel (I) nach einem der Ansprüche 1 bis 4 enthält.

13. Mittel nach einem der Ansprüche 5 bis 12, **dadurch gekennzeichnet, dass** es ein Haarfärbemittel ist.

14. Verfahren zum oxidativen Färben von Haaren, insbesondere menschlichen Haaren, **dadurch gekennzeichnet, dass** man vor der Anwendung ein Haarfärbemittel nach einem der Ansprüche 5 bis 13 mit einem Oxidationsmittel vermischt, sodann auf das Haar aufträgt, bei einer Temperatur von 15 bis 50 °C 10 bis 45 Minuten lang einwirken lässt, das Haar anschliessend mit Wasser ausspült, gegebenenfalls shampooniert und sodann trocknet.

## Claims

1. 3-Aminophenol derivatives of the formula (I), or physiologically compatible water-soluble salts thereof, 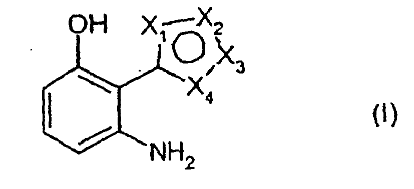 where **X1, X2, X3** and **X4,** independently of one another, are sulphur, nitrogen, N-R1, oxygen, C-R2, C-R3, C-R4 or C-R5, with the proviso that at least one and at most three of the radicals X1 to **X4** are nitrogen, N-R1, sulphur or oxygen and if there are two or more hetero atoms at most one of the radicals **X1** to **X4** is sulphur, N-R1 or oxygen;
**R1** is hydrogen, a C₁-C₆-alkyl group, a phenyl group, a (C₂-C₄)-hydroxyalkyl group or an acetyl group;
**R2, R3, R4** and **R5,** independently of one another, are hydrogen, a halogen atom, a cyano group, a C₁-C₆-alkyl group, a C₁-C₄-alkoxy group, a C₁-C₄-alkylthio group, a mercapto group, a nitro group, an amino group, a C₁-C₄-alkylamino group, a di (C₁-C₄) alkylamino group, a hydroxy(C₂-C₄)alkylamino group, a di (hydroxy(C₂-C₄)alkyl)amino group, a (dihydroxy(C₃-C₄)alkyl)amino group, a trifluoromethyl group, an acetyl group, a trifluoroacetyl group, a formyl group, a trimethylsilyl group, a (C₁-C₄)-hydroxyalkyl group or a (C₂-C₄)-dihydroxyalkyl group, or the following apply: (i) X1 = CH, X2 = CR3, X3 = CR4 and X4 = sulphur, where the radicals R3 and R4, together with the remainder of the molecule, form a fused unsubstituted benzene ring, or (ii) X4 = CH, X1 = CR2, X2 = CR3 and X3 = sulphur, where the radicals R2 and R3, together with the remainder of the molecule, form a fused unsubstituted benzene ring.

2. 3-Aminophenol derivative according to Claim 1, **characterized in that** it is chosen from 3-amino-2-(3-thienyl)phenol, 3-amino-2-(3-furyl)phenol, 3-amino-2-(pyrrol-3-yl)phenol, 3-amino-2-(1-methyl-1H-pyrrol-3-yl)phenol, 3-amino-2-(1,3-thiazol-2-yl)phenol, 3-amino-2-(1,3-thiazol-5-yl)phenol, 3-amino-2-(2-thienyl)phenol, 3-amino-2-(2-furyl)phenol, 3-amino-2-(pyrrol-2-yl)phenol, 3-amino-2-(1-methyl-1H-pyrrol-2-yl)-phenol, 3-amino-2-(2-chloro-3-thienyl)phenol, 3-amino-2-(2-methyl-3-thienyl)phenol, 3-amino-2-(benzo[b]thiophen-2-yl)phenol, 3-amino-2-(5-phenyl-2-thienyl)phenol, 3-amino-2-(2-nitro-3-thienyl)phenol, 3-amino-2-(2-amino-3-thienyl)-phenol, 3-amino-2-(2-acetyl-3-thienyl)phenol, 3-amino-2-(2-formyl-3-thienyl)phenol, 3-amino-2-(4-chloro-3-thienyl)phenol, 3-amino-2-(4-methyl-3-thienyl)phenol, 3-amino-2-(4-nitro-3-thienyl)-phenol, 3-amino-2-(4-amino-3-thienyl)phenol, 3-amino-2-(4-acetyl-3-thienyl)phenol, 3-amino-2-(4-formyl-3-thienyl)phenol, 3-amino-2-(5-chloro-3-thienyl)phenol, 3-amino-2-(5-methyl-3-thienyl)-phenol, 3-amino-2-(5-phenyl-3-thienyl)phenol, 3-amino-2-(5-nitro-3-thienyl)phenol, 3-amino-2-(5-acetyl-3-thienyl)phenol, 3-amino-2-(5-amino-3-thienyl)phenol, 3-amino-2-(5-formyl-3-thienyl)-phenol, 3-amino-2-(benzo[b]thiophen-3-yl)phenol, 3-amino-2-(5-formyl-3-furyl)phenol, 3-amino-2-(3-chloro-2-thienyl)phenol, 3-amino-2-(3-methyl-2-thienyl)phenol, 3-amino-2-(3-nitro-2-thienyl)-phenol, 3-amino-2-(3-amino-2-thienyl)phenol, 3-amino-2-(3-acetyl-2-thienyl)phenol, 3-amino-2-(3-formyl-2-thienyl)phenol, 3-amino-2-(4-chloro-2-thienyl)phenol, 3-amino-2-(4-methyl-2-thienyl)-phenol, 3-amino-2-(4-nitro-2-thienyl)phenol, 3-amino-2-(4-amino-2-thienyl)phenol, 3-amino-2-(4-acetyl-2-thienyl)phenol, 3-amino-2-(4-formyl-2-thienyl)phenol, 3-amino-2-(5-chloro-2-thienyl)-phenol, 3-amino-2-(5-methyl-2-thienyl)phenol, 3-amino-2-(5-nitro-2-thienyl)phenol, 3-amino-2-(5-amino-2-thienyl)phenol, 3-amino-2-(5-acetyl-2-thienyl)phenol, 3-amino-2-(5-formyl-2-thienyl)-phenol, 3-amino-2-(5-formyl-2-furyl)phenol, 3-amino-2-(5-nitro-1,3-thiazol-2-yl)phenol, 3-amino-2-(5-amino-1,3-thiazol-2-yl)phenol, 3-amino-2-(2-nitro-1,3-thiazol-5-yl)phenol, 3-amino-2-(2-amino-1,3-thiazol-5-yl)phenol, 3-amino-2-(3,5-dimethylisoxazol-4-yl)phenol, 3-amino-2-(3,5-dimethyl-1H-pyrazol-4-yl)phenol and 3-amino-2-(5-nitro-4H-1,2,4-triazol-3-yl)phenol, and also physiologically compatible water-soluble salts thereof.

3. 3-Aminophenol derivative according to Claim 1, **characterized in that**, in the formula (I):
(i) **X2** is sulphur, and **X1, X3** and **X4** are C-R2, C-R4 and C-R5 or (ii) **X1** is sulphur and **X2, X3** and **X4** are C-R3, C-R4 and C-R5.

4. 3-Aminophenol derivative according to one of Claims 1 to 3, **characterized in that** it is chosen from 3-amino-2-(3-thienyl)phenol, 3-amino-2-(2-thienyl)phenol, 3-amino-2-(benzo[b]thiophen-3-yl)phenol and 3-amino-2-(5-phenyl-2-thienyl)-phenol, and physiologically compatible water-soluble salts thereof.

5. Agent for dyeing keratin fibres based on a developer substance-coupler substance combination, **characterized in that** it comprises at least one 3-aminophenol derivative of the formula (I) according to one of Claims 1 to 4 as coupler substance.

6. Agent according to Claim 5, **characterized in that** the 3-aminophenol derivative of the formula (I) is present in an amount of from 0.005 to 20 per cent by weight.

7. Agent according to Claim 5 or 6, **characterized in that** the developer substance is chosen from the group consisting of 1,4-diaminobenzene, 1,4-diamino-2-methylbenzene, 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(2-thienyl)benzene, 1,4-diamino-2-(3-thienyl)benzene, 1,4-diamino-2-(pyridin-3-yl)benzene, 2,5-diaminobiphenyl, 1,4-diamino-2-methoxymethylbenzene, 1,4-diamino-2-aminomethylbenzene, 1,4-diamino-2-hydroxymethylbenzene, 1,4-diamino-2-(2-hydroxyethoxy)benzene, 2-(2-acetylamino)ethoxy-1,4-diaminobenzene, 4-phenylaminoaniline, 4-dimethylaminoaniline, 4-diethylaminoaniline, 4-dipropylaminoaniline, 4-[ethyl-(2-hydroxyethyl)amino]aniline, 4-[di(2-hydroxyethyl)amino]aniline, 4-[di(2-hydroxyethyl)amino]-2-methylaniline, 4-[(2-methoxyethyl)amino]aniline, 4-[(3-hydroxypropyl)amino]aniline, 4-[(2,3-dihydroxypropyl)amino]aniline, 1,4-diamino-2-(1-hydroxyethyl)benzene, 1,4-diamino-2-(2-hydroxyethyl)benzene, 1,4-diamino-2-(1-methylethyl)benzene, 1,3-bis[(9-aminophenyl)(2-hydroxyethyl)amino]-2-propanol, 1,4-bis[(4-aminophenyl)-amino]butane, 1,8-bis(2,5-diaminophenoxy)-3,6-dioxaoctane, 4-aminophenol, 4-amino-3-methylphenol, 4-amino-3-(hydroxymethyl)phenol, 4-amino-3-fluorophenol, 4-methylaminophenol, 4-amino-2-(aminomethyl)phenol, 4-amino-2-(hydroxymethyl)phenol, 4-amino-2-fluorophenol, 4-amino-2-[(2-hydroxyethyl)amino]methylphenol, 4-amino-2-methylphenol, 4-amino-2-(methoxymethyl)phenol, 4-amino-2-(2-hydroxyethyl)phenol, 5-aminosalicylic acid, 2,5-diaminopyridine, 2,4,5,6-tetraaminopyrimidine, 2,5,6-triamino-4-(1H)-pyrimidone, 4,5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 1-[(4-chlorophenyl)methyl]-4,5-diamino-1H-pyrazole, 4,5-diamino-1-methyl-1H-pyrazole, 2-aminophenol, 2-amino-6-methylphenol, 2-amino-5-methylphenol and 1,2,4-trihydroxybenzene.

8. Agent according to one of Claims 5 to 7, **characterized in that**, in addition to the compounds of the formula (I), it comprises at least one further known coupler substance which is chosen from the group consisting of N-(3-dimethylaminophenyl)urea, 2,6-diaminopyridine, 2-amino-4-[(2-hydroxyethyl)-amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di-[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy) benzene, 1,3-diamino-4-(3-hydroxypropoxy)-benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)-amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]aniline, 1,3-di(2,4-diaminophenoxy)-propane, di(2,4-diaminophenoxy)methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-4-methoxy-2-methylphenol, 5-[(2-hydroxyethyl)amino]-2-methylphenol, 3-[(2-hydroxyethyl)amino]phenol, 3-[(2-methoxyethyl)amino]phenol, 5-amino-2-ethylphenol, 5-amino-2-methoxyphenol, 2-(4-amino-2-hydroxyphenoxy)-ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindol, 5,6-dihydroxyindoline, 5-hydroxyindol, 6-hydroxyindol, 7-hydroxyindol and 2,3-indolinedione.

9. Agent according to one of Claims 5 to 8, **characterized in that** the developer substances and coupler substances, based on the total amount of the dye, are each present in a total amount of from 0.005 to 20 per cent by weight.

10. Agent according to one of Claims 5 to 9, **characterized in that** it additionally comprises at least one direct dye.

11. Agent according to one of Claims 5 to 10, **characterized in that** it has a pH of from 6.5 to 11.5.

12. Ready-to-use agent for the oxidative dyeing of keratin fibres, which comprises, in a medium suitable for the dyeing, at least one developer substance and at least one coupler substance, and at least one oxidizing agent, **characterized in that** it comprises at least one 3-aminophenol derivative of the formula (I) according to one of Claims 1 to 4 as coupler substance.

13. Agent according to one of Claims 5 to 12, **characterized in that** it is a hair dye.

14. Method for the oxidative dyeing of hair, in particular human hair, **characterized in that**, prior to application, a hair dye according to one of Claims 5 to 13 is mixed with an oxidizing agent, is then applied to the hair, left to act at a temperature of from 15 to 50°C for 10 to 45 minutes, and the hair is then rinsed with water, optionally shampooed and then dried.

## Revendications

1. Dérivés de 3-aminophénol de formule (I), ou leurs sels physiologiquement acceptables, solubles dans l'eau, 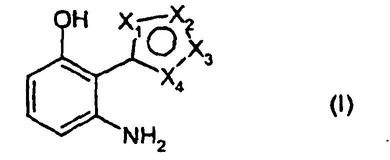 dans laquelle X1, X2, X3 et X4 représentent, indépendamment l'un de l'autre soufre, azote, N-R1, oxygène, C-R2, C-R3, C-R4 ou C-R5, à condition qu'au moins un et au maximum trois des radicaux X1 à X4 représentent azote, N-R1, soufre ou oxygène et, lorsqu'il existe plusieurs hétéroatomes, au maximum un des radicaux X1 à X4 représente soufre, N-R1 ou oxygène;
R1 représente hydrogène, un groupe alkyle en C₁ à C₆, un groupe phényle, un groupe hydroxyalkyle en C₂ à C₄ ou un groupe acétyle;
R2, R3, R4 et R5 représentent, indépendamment l'un de l'autre, hydrogène, un atome d'halogène, un groupe cyano, un groupe alkyle en C₁ à C₆, un groupe alcoxy en C₁ à C₄, un groupe (alkyle en C₁ à C₄) thio, un groupe mercapto, un groupe nitro, un groupe amino, un groupe (alkyle en C₁ à C₄) amino, un groupe di (alkyle en C₁ à C₄) amino, un groupe hydroxy (alkyle en C₂ à C₄)amino, un groupe di(hydroxy(alkyle en C₂ à C₄))amino, un groupe (dihydroxy(alkyle en C₃ à C₄))amino, un groupe trifluorométhyle, un groupe acétyle, un groupe trifluoroacétyle, un groupe formyle, un groupe triméthylsilyle, un groupe hydroxyalkyle en C₁ à C₄ ou un groupe dihydroxyalkyle en C₂ à C₄, ou (i) X1=CH, X2=CR3, X3=CR4 et X4=soufre, les radicaux R3 et R4 formant ensemble avec le reste de la molécule un cycle benzène annelé non substitué, ou (ii) X4=CH, X1=CR2, X2=CR3 et X3=soufre, les radicaux R2 et R3 formant ensemble avec le reste de la molécule un cycle benzène annelé non substitué.

2. Dérivé de 3-aminophénol selon la revendication 1, **caractérisé en ce qu'**il est choisi parmi le 3-amino-2-(3-thiényl)phénol, le 3-amino-2-(3-furyl)phénol, le 3-amino-2-(pyrrol-3-yl)phénol, le 3-amino-2-(1-méthyl-1H-pyrrol-3-yl)phénol, le 3-amino-2-(1,3-thiazol-2-yl)phénol, le 3-amino-2-(1,3-thiazol-5-yl)phénol, le 3-amino-2-(2-thiényl)phénol, le 3-amino-2-(2-furyl)-phénol, le 3-amino-2-(pyrrol-2-yl)phénol, le 3-amino-2-(1-méthyl-1H-pyrrol-2-yl)phénol, le 3-amino-2-(2-chloro-3-thiényl)-phénol, le 3-amino-2-(2-méthyl-3-thiényl)phénol, le 3-amino-2-(benzo[b]thiophén-2-yl)-phénol, le 3-amino-2-(5-phényl-2-thiényl)phénol, le 3-amino-2-(2-nitro-3-thiényl)phénol, le 3-amino-2-(2-amino-3-thiényl)phénol, le 3-amino-2-(2-acétyl-3-thiényl)phénol, le 3-amino-2-(2-formyl-3-thiényl)phénol, le 3-amino-2-(4-chloro-3-thiényl) phénol, le 3-amino-2-(4-méthyl-3-thiényl)phénol, le 3-amino-2-(4-nitro-3-thiényl)phénol, le 3-amino-2-(4-amino-3-thiényl)phénol, le 3-amino-2-(4-acétyl-3-thiényl)-phénol, le 3-amino-2-(4-formyl-3-thiényl)phénol, le 3-amino-2-(5-chloro-3-thiényl)phénol, le 3-amino-2-(5-méthyl-3-thiényl)phénol, le 3-amino-2-(5-phényl-3-thiényl)phénol, le 3-amino-2-(5-nitro-3-thiényl)phénol, le 3-amino-2-(5-acétyl-3-thiényl)phénol, le 3-amino-2-(5-amino-3-thiényl)-phénol, le 3-amino-2-(5-formyl-3-thiényl) phénol, le 3-amino-2-(benzo[b]-thiophén-3-yl)-phénol, le 3-amino-2-(5-formyl-3-furyl)phénol, le 3-amino-2-(3-chloro-2-thiényl)phénol, le 3-amino-2-(3-méthyl-2-thiényl)phénol, le 3-amino-2-(3-nitro-2-thiényl)phénol, le 3-amino-2-(3-amino-2-thiényl)phénol, le 3-amino-2-(3-acétyl-2-thiényl)-phénol, le 3-amino-2-(3-formyl-2-thiényl)phénol, le 3-amino-2-(4-chloro-2-thiényl)phénol, le 3-amino-2-(4-méthyl-2-thiényl)-phénol, le 3-amino-2-(4-nitro-2-thiényl)phénol, le 3-amino-2-(4-amino-2-thiényl)phénol, le 3-amino-2-(4-acétyl-2-thiényl)phénol, le 3-amino-2-(4-formyl-2-thiényl)phénol, le 3-amino-2-(5-chloro-2-thiényl)phénol, le 3-amino-2-(5-méthyl-2-thiényl)phénol, le 3-amino-2-(5-nitro-2-thiényl)phénol, 3-amino-2-(5-amino-2-thiényl)phénol, le 3-amino-2-(5-acétyl-2-thiényl)phénol, le 3-amino-2-(5-formyl-2-thiényl)phénol, le 3-amino-2-(5-formyl-2-furyl)phénol, 3-amino-2-(5-nitro-1,3-thiazol-2-yl)phénol, le 3-amino-2-(5-amino-1,3-thiazol-2-yl)-phénol, le 3-amino-2-(2-nitro-1,3-thiazol-5-yl)phénol, le 3-amino-2-(2-amino-1,3-thiazol-5-yl)phénol, le 3-amino-2-(3,5-diméthylisoxazol-4-yl)-phénol, le 3-amino-2-(3,5-diméthyl-1H-pyrazol-4-yl)phénol et le 3-amino-2-(5-nitro-4H-1,2,4-triazol-3-yl)phénol ainsi que leurs sels physiologiquement acceptables solubles dans l'eau.

3. Dérivé de 3-aminophénol selon la revendication 1, **caractérisé en ce que**, dans la formule (I) :
(i) X2 représente soufre, et X1, X3 et X4 représentent C-R2, C-R4 et C-R5 ou (ii) X1 représente soufre et X2, X3 et X4 représentent C-R3, C-R4 et C-R5.

4. Dérivé de 3-aminophénol selon l'une quelconque des revendications 1 à 3, **caractérisé en ce qu'**il est choisi parmi le 3-amino-2-(3-thiényl)phénol, le 3-amino-2-(2-thiényl)phénol, le 3-amino-2-(benzo[b]-thiophén-3-yl)-phénol et le 3-amino-2-(5-phényl-2-thiényl)-phénol ainsi que leurs sels physiologiquement acceptables solubles dans l'eau.

5. Agent pour la teinture de fibres kératiniques à base d'une combinaison substance de développement-substance de couplage, **caractérisé en ce qu'**il contient, comme substance de couplage, au moins un dérivé de 3-aminophénol de formule (I) selon l'une quelconque des revendications 1 à 4.

6. Agent selon la revendication 5, **caractérisé en ce que** le dérivé de 3-aminophénol de formule (I) est contenu en une quantité de 0,005 à 20% en poids.

7. Agent selon la revendication 5 ou 6, **caractérisé en ce que** la substance de développement est choisie dans le groupe constitué par le 1,4-diamino-benzène, le 1,4-diamino-2-méthyl-benzène, le 1,4-diamino-2,6-diméthyl-benzène, le 1,4-diamino-3,5-diéthyl-benzène, le 1,4-diamino-2,5-diméthyl-benzène, le 1,4-diamino-2,3-diméthyl-benzène, le 2-chloro-1,4-diaminobenzène, le 1,4-diamino-2-(2-thiényl)benzène, le 1,4-diamino-2-(3-thiényl)benzène, le 1,4-diamino-2-(pyridin-3-yl)benzène, le 2,5-diamino-biphényle, le 1,4-diamino-2-méthoxyméthyl-benzène, le 1,4-diamino-2-amino-méthyl-benzène, le 1,4-diamino-2-hydroxyméthyl-benzène, le 1,4-diamino-2-(2-hydroxyéthoxy)-benzène, le 2-(2-(acétylamino)éthoxy)-1,4-diamino-benzène, la 4-phénylamino-aniline, la 4-diméthylamino-aniline, la 4-diéthylamino-aniline, la 4-dipropylamino-aniline, la 4-[éthyl(2-hydroxyéthyl)amino]-aniline, la 4-[di(2-hydroxyéthyl)amino]-aniline, la 4-[di(2-hydroxyéthyl)amino]-2-méthylaniline, la 4-[(2-méthoxyéthyl)amino]-aniline, la 4-[(3-hydroxypropyl)amino]-aniline, la 4-[(2,3-dihydroxypropyl)amino]-aniline, le 1,4-diamino-2-(1-hydroxyéthyl)-benzène, le 1,4-diamino-2-(2-hydroxyéthyl)-benzène, le 1,4-diamino-2-(1-méthyléthyl)-benzène, le 1,3-bis[(4-aminophényl)(2-hydroxyéthyl)amino]-2-propanol, le 1,4-bis[(4-aminophényl)amino]-butane, le 1,8-bis(2,5-diaminophénoxy)-3,6-dioxaoctane, le 4-amino-phénol, le 4-amino-3-méthyl-phénol, le 4-amino-3-(hydroxyméthyl)-phénol, le 4-amino-3-fluoro-phénol, le 4-méthylaminophénol, le 4-amino-2-(aminométhyl)-phénol, le 4-amino-2-(hydroxyméthyl)-phénol, le 4-amino-2-fluorophénol, le 4-amino-2-[(2-hydroxyéthyl)-amino]méthylphénol, le 4-amino-2-méthyl-phénol, le 4-amino-2-(méthoxyméthyl)-phénol, le 4-amino-2-(2-hydroxyéthyl)-phénol, l'acide 5-amino-salicylique, la 2,5-diaminopyridine, la 2,4,5,6-tétraamino-pyrimidine, la 2,5,6-triamino-4-(1H)-pyrimidone, le 4,5-diamino-1-(2-hydroxyéthyl)-1H-pyrazole, le 4,5-diamino-1-(1-méthyléthyl)-1H-pyrazole, le 4,5-diamino-1-[(4-méthylphényl)méthyl]-1H-pyrazole, le 1-[(4-chlorophényl)méthyl]-4,5-diamino-1H-pyrazole, le 4,5-diamino-1-méthyl-1H-pyrazole, le 2-amino-phénol, le 2-amino-6-méthyl-phénol, le 2-amino-5-méthyl-phénol et le 1,2,4-trihydroxy-benzène.

8. Agent selon l'une quelconque des revendications 5 à 7, **caractérisé en ce qu'**il contient, en plus des composés de formule (I) au moins une autre substance de couplage connue, qui est choisie dans le groupe constitué par la N-(3-diméthylamino-phényl)-urée, la 2,6-diamino-pyridine, le 2-amino-4-[(2-hydroxyéthyl)amino]-anisole, 2,4-diamino-1-fluoro-5-méthyl-benzène, le 2,4-diamino-1-méthoxy-5-méthyl-benzène, le 2,4-diamino-1-éthoxy-5-méthyl-benzène, le 2,4-diamino-1-(2-hydroxyéthoxy)-5-méthyl-benzène, le 2,4-di[(2-hydroxyéthyl)amino]-1,5-diméthoxy-benzène, la 2,3-diamino-6-méthoxy-pyridine, la 3-amino-6-méthoxy-2-(méthylamino)-pyridine, la 2,6-diamino-3,5-diméthoxy-pyridine, la 3,5-diamino-2,6-diméthoxy-pyridine, le 1,3-diamino-benzène, le 2,4-diamino-1-(2-hydroxyéthoxy)-benzène, le 1,3-diamino-4-(2,3-dihydroxypropoxy)-benzène, le 1,3-diamino-4-(3-hydroxypropoxy)-benzène, le 1,3-diamino-4-(2-méthoxyéthoxy)-benzène, le 2,4-diamino-1,5-di(2-hydroxyéthoxy)-benzène, le 1-(2-aminoéthoxy)-2,4-diamino-benzène, le 2-amino-1-(2-hydroxyéthoxy)-4-méthylamino-benzène, l'acide 2,4-diaminophénoxyacétique, la 3-[di(2-hydroxyëthyl)amino]-aniline, le 4-amino-2-di[(2-hydroxyéthyl)amino]-1-éthoxy-benzène, le 5-méthyl-2-(1-méthyléthyl)-phénol, la 3-[(2-hydroxyéthyl)amino]-aniline, la 3-[(2-aminoéthyl)amino]-aniline, le 1,3-di(2,4-diaminophénoxy)-propane, le di(2,4-diaminophénoxy)-méthane, le 1,3-diamino-2,4-diméthoxy-benzène, le 2,6-bis(2-hydroxyéthyl)amino-toluène, le 4-hydroxyindole, le 3-diméthylaminophénol, le 3-diéthylamino-phénol, le 5-amino-2-méthyl-phénol, le 5-amino-4-fluoro-2-méthyl-phénol, le 5-amino-4-méthoxy-2-méthyl-phénol, le 5-amino-4-éthoxy-2-méthyl-phénol, le 3-amino-2,4-dichloro-phénol, le 5-amino-2,4-dichlorophénol, le 3-amino-2-méthyl-phénol, le 3-amino-2-chloro-6-méthyl-phénol, le 3-amino-phénol, le 2-[(3-hydroxyphényl)-amino]-acétamide, le 5-[(2-hydroxyéthyl)amino]-4-méthoxy-2-méthyl-phénol, le 5-[(2-hydroxyéthyl)amino]-2-méthyl-phénol, le 3-[(2-hydroxyéthyl)amino]-phénol, le 3-[(2-méthoxyéthyl)-amino]-phénol, le 5-amino-2-éthyl-phénol, le 5-amino-2-méthoxy-phénol, le 2-(4-amino-2-hydroxyphénoxy)-éthanol, le 5-[(3-hydroxypropyl)amino]-2-méthyl-phénol, le 3-[(2,3-dihydroxypropyl)-amino]-2-méthyl-phénol, le 3-[(2-hydroxyéthyl)amino]-2-méthyl-phénol, la 2-amino-3-hydroxy-pyridine, la 2,6-dihydroxy-3,4-diméthylpyridine, le 5-amino-4-chloro-2-méthyl-phénol, le 1-naphtol, le 2-méthyl-1-naphtol, le 1,5-dihydroxy-naphtalène, le 1,7-dihydroxy-naphtalène, le 2,3-dihydroxy-naphtalène, le 2,7-dihydroxynaphthalène, le 2-méthyl-1-naphtol-acétate, le 1,3-dihydroxy-benzène, le 1-chloro-2,4-dihydroxy-benzène, le 2-chloro-1,3-dihydroxy-benzène, le 1,2-dichloro-3,5-dihydroxy-4-méthyl-benzène, le 1,5-dichloro-2,4-dihydroxy-benzène, le 1,3-dihydroxy-2-méthyl-benzène, le 3,4-méthylènedioxy-phénol, la 3,4-méthylènedioxy-aniline, le 5-[(2-hydroxyéthyl)amino]-1,3-benzodioxol, le 6-bromo-1-hydroxy-3,4-méthylènedioxy-benzène, l'acide 3,4-diamino-benzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxy-indole, la 5,6-dihydroxy-indoline, le 5-hydroxy-indole, le 6-hydroxy-indole, le 7-hydroxy-indole et la 2,3-indolinedione.

9. Agent selon l'une quelconque des revendications 5 à 8, **caractérisé en ce que** les substances de développement et les substances de couplage, par rapport à la quantité totale de l'agent de teinture, sont à chaque fois contenus en une quantité totale de 0,005 à 20% en poids.

10. Agent selon l'une quelconque des revendications 5 à 9, **caractérisé en ce qu'**il contient en outre au moins un colorant montant directement sur la fibre.

11. Agent selon l'une quelconque des revendications 5 à 10, **caractérisé en ce qu'**il présente un pH de 6,5 à 11,5.

12. Agent prêt à l'emploi pour la teinture par oxydation de fibres kératiniques qui contient, dans un agent approprié pour la teinture, au moins une substance de développement et au moins une substance de couplage ainsi qu'au moins un oxydant, **caractérisé en ce qu'**il contient comme substance de couplage au moins un dérivé de 3-aminophénol de formule (I) selon l'une quelconque des revendications 1 à 4.

13. Agent selon l'une quelconque des revendications 5 à 12, **caractérisé en ce qu'**il s'agit d'un agent de teinture des cheveux.

14. Procédé pour la teinture par oxydation de cheveux, en particulier de cheveux humains, **caractérisé en ce qu'**on mélange, avant l'utilisation, un agent de teinture pour cheveux selon l'une quelconque des revendications 5 à 13 avec un oxydant, on l'applique ensuite sur les cheveux, on laisse agir à une température de 15 à 50°C pendant 10 à 45 minutes, on rince ensuite les cheveux avec de l'eau, on les shampouine le cas échéant puis on les sèche.
